# EUROPEAN PATENT SPECIFICATION

(11) **EP 1 053 029 B1**
(45) Date of publication and mention of the grant of the patent: **10.09.2003**
(21) Application number: 99958396.6
(22) Date of filing: 06.12.1999
(51) Int. Cl.: A61L 15/28, A61L 15/32, A61L 15/44, A61L 15/22, A61L 31/00

(54) **STERILE COMPLEX OF THERAPEUTIC PEPTIDE BOND TO A POLYSACCHARIDE**
STERILER KOMPLEX EINES THERAPEUTISCHEN PEPTIDS AN EIN POLYSACCHARID
COMPLEXE STERILE DE PEPTIDE THERAPEUTIQUE SE LIANT A UN POLYSACCHARIDE

(30) Priority: 07.12.1998 GB 9826897
(43) Date of publication of application: 22.11.2000
(73) Proprietor: Johnson & Johnson Medical Ltd., Edinburgh EH2 4NH (GB)
(72) Inventor: CULLEN, Breda, Skipton North Yorkshire BD23 1HR (GB); SILCOCK, Derek, Skipton North Yorkshire BD23 1QP (GB); VAN LEEUWEN, Peter, D-22397 Hamburg (DE); HARVEY, Wilson, Skipton North Yorkshire BD23 3DW (GB)
(74) Representative: James, Anthony Christopher W.P.
(86) International application number: GB9904094
(87) International publication number: WO00033893

(56) References cited:
- EP-A- 0 238 839
- EP-A- 0 267 015
- EP-A- 0 312 208
- WO-A-91/04019
- WO-A-95/18635
- CA-A- 2 094 658
- GB-A- 2 257 909
- GB-A- 2 314 840
- GB-A- 2 314 842

## Description

The present invention relates to sterile compositions comprising one or more therapeutic peptides. The present invention also relates to methods for the preparation of such compositions, and to methods for the preparation of sterile therapeutic peptides.

Natural and synthetic peptide compounds are assuming increasing importance as active agents in therapy. There is particular interest in the use of growth factors such as epidermal growth factor (EGF), fibroblast growth factor (FGF), bone morphogenetic protein, platelet derived growth factor (PDGF) and transforming growth factors (TGF), especially for the treatment of wounds. Growth factors are becoming available in substantial quantities from recombinant DNA synthetic techniques, which raises a need for sterile formulations for administration of the growth factors.

A feature of peptide-based medicaments is that most peptides are not suitable for enteral administration, because they are broken down in the digestive tract. Therefore, most peptide medicaments must be administered parenterally, and in particular by topical or intravenous administration. For such methods of administration, it is essential that the peptide composition must be sterile. That is to say, the composition must be substantially free from viable microorganisms, viruses and DNA fragments.

A difficulty arises when attempting to sterilise compositions comprising therapeutic peptides. Conventional sterilisation methods, such as heating in an autoclave or gamma-irradiation, frequently degrade the biological activity of therapeutic peptides, especially if the sterilisation is carried out in the presence of water. Chemical sterilants are also unsuitable because of the sensitivity of peptides to oxidising agents, changes in pH, changes in ionic strength or changes in temperature. Sterilising with ethylene oxide is also unsuitable, because of reactivity of ethylene oxide with some peptides, and further because of unacceptable residues of ethylene oxide and harmful reaction products thereof in the products.

In the past, peptide based medicaments have been formulated from sterile starting materials under aseptic conditions. Such aseptic manufacture is relatively expensive and difficult to perform. Peptide-containing solutions have also been sterilised by ultrafiltration, but this method is not suitable for compositions containing macromolecules, or for solid or semi-solid compositions.

EP-A-0238839 describes wound dressings comprising an absorbent cellulosic sheet impregnated with an enzyme such as trypsin. The wound dressings are prepared and dried under aseptic conditions, apparently without subsequent sterilisation.

EP-A-0312208 describes gel formulations containing polypeptide growth factors having human mitogenic or angiogenic activity. The gel formulations are suitable for topical application to wounds, and include cellulose derivatives that are said to be capable of stabilising the polypeptide growth factors against the loss of biological activity that normally takes place when growth factors are stored in aqueous solution. Methyl cellulose and hydroxyalkyl cellulose derivatives are preferred. The gels are sterilised before addition of the peptide growth factors, and addition of the peptide growth factors is then carried out under sterile conditions. It therefore appears that there is no sterilisation of the compositions after formulation. Similar compositions are described in EP-A-0267015.

EP-A-0308238 describes stable freeze-dried compositions comprising polypeptide growth factors. The freeze-drying stabilises the growth factors against loss of activity by hydrolysis in the water. The freeze-dried compositions include extenders such as water soluble or water swellable polymers, including cellulose derivatives. The specification discloses autoclave sterilisation of the starting materials other than growth factors, and sterile filtering of the growth factors, in order to produce a sterile freeze-dried product. The application apparently does not contemplate sterilising the final freeze-dried peptide composition.

WO95/02411 describes aqueous compositions comprising a polypeptide growth factor which exhibit long-term biochemical stability of the growth factor at 4°C. The growth factor is stabilized by providing the composition with a low pH in the range 2.8 to 3.8. However, sterilisation of compositions containing the growth factors is not described in this specification.

EP-A-0437095 describes a process for preparing a neutralised oxidised cellulose product (nORC) by treatment of ORC with a solution of sodium acetate or the like. The neutralised product may be impregnated with acid-sensitive haemostatic agents, such as thrombin, to enhance its haemostatic properties, or with acid-sensitive adhesion preventive agents, such as tissue plasminogen activator (t-PA) to enhance its adhesion-prevention properties. In other embodiments, the cloth is impregnated with medicaments, such as growth factors.

Both ORC and n-ORC can be sterilised by gamma-irradiation, and EP-A-0437095 also teaches that n-ORC having heparin or heparin fragments complexed thereto can also be sterilised by gamma-irradiation. However, the reference does not describe sterilisation of ORC or n-ORC having a peptide associated therewith.

EP-A-0562864 describes composite wound dressing materials comprising a matrix of collagen sponge, a substructure of a second bioabsorbable polymer which may for example be dispersed fibre of oxidised regenerated cellulose (ORC), and an active agent which may be a peptide. The active agent can be provided in the matrix, or in the substructure, or in both. The composite sponge materials can be packaged and sterilised. The materials described in this reference are inherently inhomogeneous. They comprise a substructure of fibres, films or flakes providing phasic release of active agents and, preferably, directional cellular ingrowth.

WO98/00180 describes the use of complexes of ORC with collagen for chronic wound healing. The specification notes that ORC forms stable complexes with cell growth factors.

US-A-5730933 describes a method for sterilising biologically active peptides by gamma ray or electron beam radiation without loss of biological activity. The method comprises the steps of: forming a mixture comprising the biologically active peptide and an extraneous protein such as gelatin, freezing or freeze-drying the mixture, followed by irradiating the mixture. The presence of extraneous protein is said to stabilise the peptide against loss of biological activity. The mixture may further contain a free radical scavenger to improve the radiation stability still further. There is no suggestion that polysaccharides can achieve stabilisation of the peptides during irradiation.

It is an object of the present invention to provide an improved method for sterilising biologically active peptides such that the biological activity of the peptides is retained after sterilising.

The present invention is based on the surprising finding that peptide therapeutic agents, and in particular growth factors, can be stabilised against loss of biological activity during sterilisation with ionising radiation if the peptides are formulated with and effective amount of a selected polysaccharide prior to sterilisation.

In a first aspect, the present invention provides a sterile composition comprising a complex of a therapeutic peptide and a polysaccharide selected from the group consisting of cellulose derivatives, chitin, chitosans, galactomannans, and mixtures thereof, wherein said complex has been sterilised with ionising radiation.

The peptide may be any therapeutically active peptide, including hormones, antibodies, antibody fragments, natural and synthetic peptide antigens and antigen fragments. Suitable peptides include oxytocin, vasopressin, corticotrophin, aprotinin, calcitonin, prolactin, inhibitin, interferons, somatostatin, insulin, glucagon, auricular natriuretic factor (ANF), endorphin, a renin inhibitor, luteinizing hormone-releasing hormone (LHRH), growth hormone releasing hormone (GHRH), Peptide T or synthetic homologs or analogs thereof. Preferably, the peptide is therapeutically active to promote wound healing. Suitable peptides include haemostatic agents such as thrombin, and anti-adhesion agents such as t-PA. Also included are therapeutic fragments of natural proteins, such as collagen fragments. The invention is suitable for both water-soluble and water-insoluble peptides. The peptide may have any molecular weight, for example in the range 1000-100000, preferably 4000-60000.

Preferably, the peptide comprises a growth factor, more preferably a growth factor having human mitogenic or angiogenic activity. More preferably, the growth factor is selected from the group consisting of fibroblast growth factor (FGF), platelet derived growth factor (PDGF), transforming growth factors (TGF-α, TGF-β₁, TGF-β₂), nerve growth factors (NGF-α, NGF-β), epidermal growth factor (EGF), bone morphogenetic protein, insulin-like growth factor (IGF-I or IGF-II), and mixtures thereof.

The present inventors have found certain groups of polysaccharides, both natural and chemically modified, are effective to stabilise peptides against sterilisation by ionising radiation. The effective polysaccharides that have been identified are: (a) cellulose derivatives; (b) chitin and chitosans, and (c) galactomannans. Other polysaccharides such as sodium hyaluronate, pectin, β-glucan or calcium alginate are not suitable, either because they do not protect the biological activity of peptides during sterilisation, or because they are not expected to release biologically active peptide in the body. The sterile products resulting from such sterilisation of peptide complexes are identifiable by their high biological activity and by characteristic ionisation breakdown fragments present in the compositions.

The term "cellulose derivative" refers to any chemically modified cellulose. Preferably, the modified cellulose includes at least one carboxylate group for each 100 saccharide residues, and more preferably it comprises a carboxymethyl cellulose, an oxidised cellulose, or salts thereof.

Oxidised cellulose is prepared by oxidation of some of the -CH₂OH groups on cellulose to carboxylate groups by treatment with nitrogen dioxide or another oxidizing agent. Oxidised cellulose can be used to prepare bioresorbable and absorbent matrices capable of convenient application to tissue surfaces. Oxidised regenerated cellulose (ORC) fabrics are especially preferred, since they can be formed by oxidative treatment of regenerated cellulose fabrics, such as rayon-type fabrics. Two examples of such ORC fabrics are INTERCEED (Registered Trade Mark of Johnson & Johnson Medical, Inc.) and SURGICEL (Registered Trade Mark of Johnson & Johnson Medical, Inc.). The INTERCEED fabric is used as an adhesion barrier in surgery. The SURGICEL fabric is used as a haemostat in surgery and in other applications. ORC offers important advantages over unmodified cellulose fabrics, in particular bioresorbability *in vivo,* and haemostatic properties.

Suitable ORC may be prepared by the method of US-A-3122479 or from commercially available INTERCEED® or SURGICEL® fabrics, or in powdered or fibrous form by milling such fabrics. Neutralised ORC may be prepared as described in EP-A-0437095. Lower molecular weight ORC fragments, including water-soluble fragments, may be prepared by alkali hydrolysis of ORC as described in WO98/00446.

Also effective in the present invention are chitin, chitosan and all intermediate partially deacylated chitosans.

Also effective in the present invention are galactomannans, that is to say polysaccharides comprising both galactose and mannose residues. Preferred galactomannans include guar gum and locust bean gum.

The complex of therapeutically active peptide with polysaccharide used in the present invention may further comprise other natural or semi-synthetic biocompatible polymers in admixture with the above-identified polysaccharides. Suitable additional biopolymers include: structural proteins such as collagen, fibrin and laminin; modified structural proteins such as atelocollagen or pepsin-solubilised collagen; further polysaccharides such as cellulose, starch, alginates, modified starches or mucopolysaccharides, and mixtures thereof. Preferably, the additional biopolymers are selected from the group consisting of native and modified structural proteins and polyanionic polysaccharides. The term "anionic polysaccharide" encompasses mucopolysaccharides such as heparin, hyaluronic acid, heparan sulphate, chondroitin sulphate and fragments and salts thereof.

The term structural protein in this specification refers to collagen and other structural proteins such as fibrin or laminin. These are not therapeutically active peptides in the normal sense of the word. Complexes between such structural proteins and alginate are described in US-A-4614794. Complexes between such structural proteins and ORC are described in GB-A-2314842. Such complexes provide advantages in wound healing applications, especially for the treatment of chronic wounds. Preferably, the structural protein consists essentially of native fibrous collagen.

The composition according to this aspect of the invention preferably contains a therapeutically effective amount of the peptide for the intended use. Typically, in the case of compositions for topical application to wounds, this would be in the range of 0.1 to 10,000 ppm by weight, more preferably 1 to 1,000 ppm by weight. Preferably, the weight ratio of the therapeutic peptide to the polysaccharide in the composition is from 1:10⁶ to 1:10, more preferably from 1:10⁵ to 1:100, and most preferably from 1:10⁴ to 1:1000.

The therapeutic peptide is complexed to the polysaccharide. That is to say, a substantial fraction of the molecules of the therapeutic peptides are associated with molecules of the polysaccharide by covalent, ionic or Van der Waals bonding. This can be achieved, for example, by coating a thin layer onto a porous or textile polysaccharide substrate, or by intimate mixing of the peptide and the polysaccharide. Preferably, the therapeutic peptide is not covalently bonded to the polysaccharide.

It is a further advantage of the polysaccharides used in the present invention that the bound peptide can be released from the complex quite readily. Preferably, at least 20% and more preferably at least 40 % of the peptide biological activity (based on the activity of the peptide before binding) can be released from the complex into aqueous solution simply by soaking the complex in water or serum at 20 °C for 24 hours.

The complex is typically formed by intimate mixing of the therapeutic peptide and the polysaccharide at the molecular level, for example by co-dispersing the therapeutic peptide and the polysaccharide in a solvent, followed by removal of the solvent.

Preferably, the complex of therapeutic peptide and polysaccharide is in the form of solid or colloidal particles dispersed in a pharmaceutically acceptable carrier. Preferably, the particles are less than 50 µm in diameter, more preferably less than 10 µm, and most preferably less than 2 µm in diameter. The pharmaceutically acceptable carrier may be a solid surface or a solid matrix. However, preferably, the carrier is an aqueous liquid or gel in which the therapeutic peptide and polysaccharide are dispersed. In certain preferred embodiments, the sterile composition according to the present invention is a viscous liquid or a gel preferably having a viscosity of at least 100 Ns/m at 20°C, and suitable for topical administration to the human or animal body, especially to wounds.

In other preferred embodiments, the sterile composition according to the present invention is a liquid, for example a buffered saline, containing the therapeutic peptide and the polysaccharide, and suitable for intravenous administration to the human or animal body.

In other preferred embodiments, the sterile composition of the present invention may comprise a solid pharmaceutical carrier, with the therapeutic peptide and the polysaccharide coated onto a surface of the carrier. Preferably, the solid carrier is a woven or nonwoven fabric, a polymer film, or a web, suitable for application to a surface of a wound. Particularly preferred solid carriers are bioabsorbable solid carriers, especially woven or nonwoven ORC cloth. These embodiments may, for example, be manufactured by coating the therapeutic peptide onto a solid carrier of the polysaccharide (or a solid carrier coated with the polysaccharide), drying to form the peptide/polysaccharide complex at the surface of the solid carrier, and sterilising.

Preferably, the sterile compositions according to the present invention are sterile packaged. That is to say, they are preferably packaged in a sterile microorganism-impermeable container.

In a second aspect, the present invention provides a process for the preparation of a sterile therapeutic composition comprising the steps of:
providing a complex of a therapeutic peptide with a polysaccharide; sterilising the complex; and dispersing the complex in or on a pharmaceutically acceptable carrier, said polysaccharide being selected from the group consisting of cellulose derivatives, chitin, chitosans, galactomannans, and mixtures thereof.

The preferred therapeutic peptide, polysaccharide and pharmaceutically acceptable carrier are as hereinbefore defined with respect to the first aspect of the invention.

Preferably, the complex is formed by dispersing the therapeutic peptide and the polysaccharide in a solvent, followed by removing the solvent to leave the complex. Preferably, the solvent is an aqueous solvent, and more preferably it consists essentially of water. Preferably, the solvent is removed by freeze drying or solvent drying. This generally results in a highly porous polysaccharide sponge having the therapeutic peptide contacted thereto. This material can then be milled for subsequent dispersal in the pharmaceutically acceptable carrier.

In other preferred methods, the polysaccharide is in the form of a woven or nonwoven fabric, a polymer film, a web or a sponge, and is coated with the therapeutic peptide, preferably by dipping the polysaccharide into a solution of the therapeutic peptide. The solvent is then removed by evaporation to leave the polysaccharide carrier having the therapeutic peptide complex to the surface thereof.

The step of sterilising the complex is preferably carried out on therapeutic peptide/polysaccharide complex, preferably containing less than 10% by weight of water, more preferably less than 4%, and most preferably substantially anhydrous. The sterilising may be carried out by any of the conventional methods, including autoclaving and treatment with ethylene oxide. Preferably, the sterilising is carried out by treatment with a gas plasma, for example in a STERAD (registered trade mark) machine. Preferably, the sterilising is carried out with ionising radiation such as ultraviolet light, electron beams or gamma irradiation. More preferably, the ionising radiation is gamma radiation, most preferably at a sterilising dosage of at least 1 to 50 kGy of cobalt-60 radiation, preferably 20-30 kGy. That is to say, preferably with about 1 to 3 mRad of gamma radiation. Surprisingly, the irradiation of the therapeutic peptides complexed to the selected polysaccharide does not substantially degrade the activity of the therapeutic peptide, despite the well known tendency of peptides to be degraded by gamma-irradiation. Without wishing to be bound by any theory, the surprising stability of the therapeutic peptides complexed to these polysaccharides may be due to the polysaccharides acting as a trap for free radicals produced by the radiation.

The complex of biologically active peptide and polysaccharide used in the present invention may further comprise a free-radical scavenger. Scavengers suitable for use comprise antioxidants such as tocopherol, citric acid, butylated hydroxyanisole, butylated hydroxytoluene, tertiary butyl hydroquinone, propylgallate, ascorbate, and other antioxidants that are generally recognised as safe for food and drug applications. Preferably, the complex comprises about 0.01 to 10 weight percent of the free radical scavenger, more preferably from 0.1 to 5 weight percent of the free-radical scavenger.

Preferably, the step of dispersing is carried out under aseptic conditions after the step of sterilising. This is especially useful for sterilising dry complexes of therapeutic peptides with the polysaccharide, followed by dispersing the sterilised complex in a liquid, gel or semi-solid carrier under aseptic conditions to form the sterile therapeutic composition.

In a third aspect, the present invention provides a process for the preparation of a sterile therapeutic peptide comprising the steps of: providing a therapeutic peptide; forming a complex between the therapeutic peptide and a polysaccharide; sterilising the complex; followed by separating the therapeutic peptide from the complex under aseptic conditions to obtain the sterile therapeutic peptide, said polysaccharide being selected from the group consisting of cellulose derivatives, chitin, chitosans, galactomannans, and mixtures thereof.

This aspect of the invention again makes use of the remarkable ability of polysaccharides to stabilise therapeutic peptides against decomposition under standard sterilising conditions. The preferred therapeutic peptides, polysaccharides and sterilisation methods are as specified above for the first and second aspects of the present invention.

Preferably, the therapeutic peptide is separated from the sterilised complex simply by solvent extraction, preferably by an aqueous solvent. Thus, for example, a complex comprising a soluble therapeutic peptide with a water-insoluble polysaccharide can be separated simply by dispersing or suspending the complex in water, followed by filtration or micro-filtration to separate the dissolved therapeutic peptide from the polysaccharide. It is a particlular advantage of ORC and chitosans that they appear to release bound peptides readily in aqueous solution. Other conventional aseptic separation methods, such as chromatography, may of course be used. The sterile therapeutic peptide may be further purified by chromatography or the like, and may be concentrated by freeze drying. Preferably, the sterile therapeutic peptide is substantially pure.

To summarise, the present invention stabilises therapeutic peptides against decomposition under standard sterilisation conditions by forming a complex between the therapeutic peptide and a selected polysaccharide. Preferably, the complex is anhydrous e.g. a freeze-dried complex. The resulting sterile complex can be formulated into conventional therapeutic products for topical or parenteral administration.

It can thus be seen that the present invention provides the use of a polysaccharide selected from the group consisting of cellulose derivatives, chitin, chitosans, galactomannans, and mixtures thereof, for the preparation of a complex with a therapeutic peptide for use in the sterilisation of the therapeutic peptide with ionising radiation with reduced loss of biological activity. Preferably, at least 20% of the original biological activity of the peptide can be recovered from the sterilised complex, and more preferably at least 40% thereof.

Alternatively, the present invention provides a method for the sterilisation of a biologically active peptide with reduced loss of biological activity, wherein the method comprises forming a complex between the peptide and a polysaccharide selected from the group consisting of cellulose derivatives, chitin, chitosans, galactomannans, and mixtures thereof, and sterilising the complex using ionising radiation.

Embodiments of the present invention will now be described further, with reference to the accompanying drawings, in which:

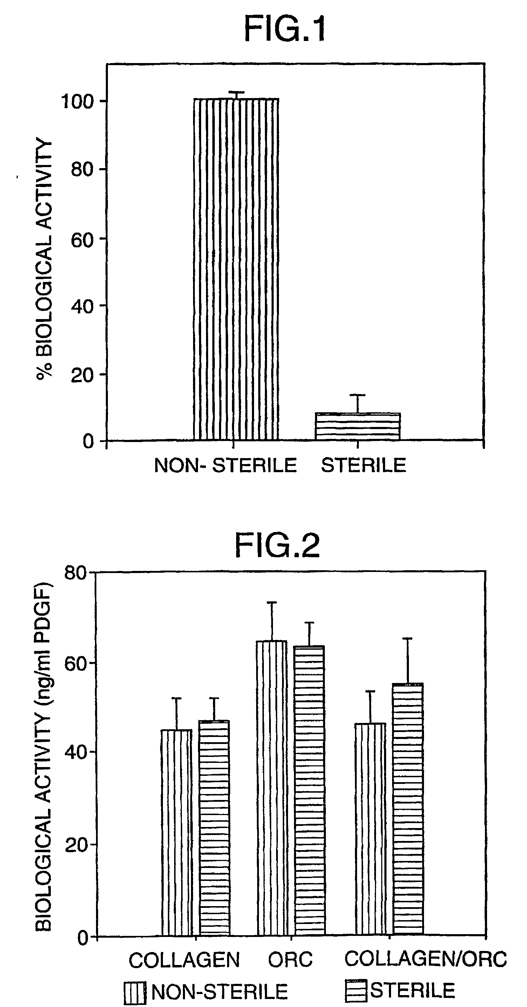

Figure 1 shows a chart of the activity of freeze-dried PDGF (comparative example) before and after sterilisation with gamma-irradiation;
Figure 2 shows a chart of the activity of PDGF complexed with (i) collagen only (ii) ORC only, and (iii) a collagen/ORC composite, before (unshaded bars) and after (shaded bars) sterilisation with gamma-irradiation.

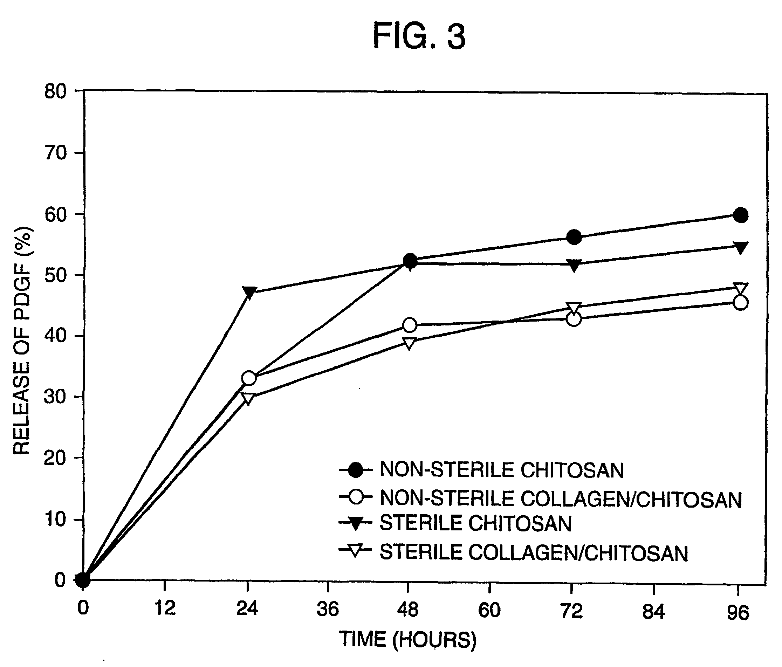

Figure 3 shows a chart of measured PDGF activity in solution against extraction time for the following complexes: (i) PDGF complexed to chitosan without gamma irradiation; (ii) PDGF complexed to a collagen/chitosan 55:45 mixture; (iii) PDGF complexed to chitosan and sterilised by gamma-irradiation; and (iv) PDGF complexed to a collagen/chitosan 55:45 blend and sterilised by gamma-irradiation.

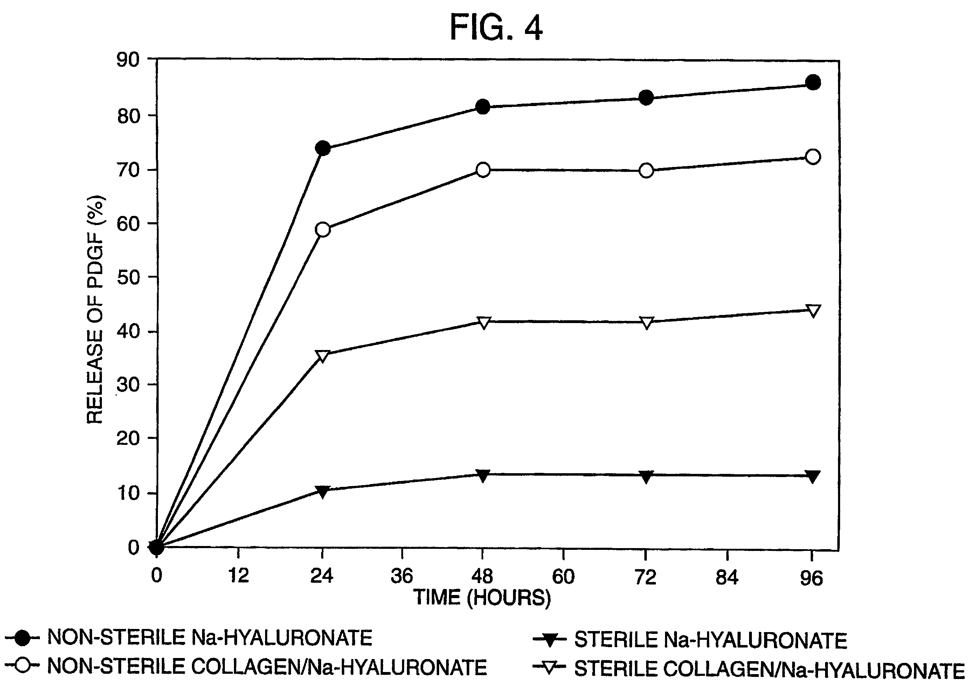

Figure 4 shows a chart of measured PDGF activity in solution against extraction time for the following complexes: (i) PDGF complexed to sodium hyaluronate (NaHA) without gamma irradiation; (ii) PDGF complexed to a collagen/NaHA 55:45 mixture; (iii) PDGF complexed to NaHA and sterilised by gamma-irradiation; and (iv) PDGF complexed to a collagen/NaHA 55:45 blend and sterilised by gamma-irradiation.

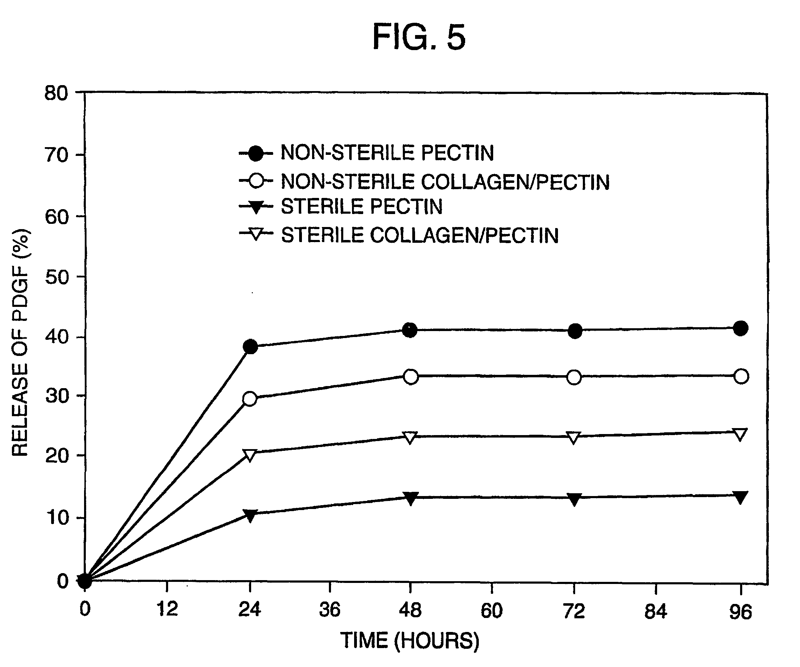

Figure 5 shows a chart of measured PDGF activity in solution against extraction time for the following complexes: (i) PDGF complexed to pectin without gamma irradiation; (ii) PDGF complexed to a collagen/pectin 55:45 mixture; (iii) PDGF complexed to pectin and sterilised by gamma-irradiation; and (iv) PDGF complexed to a collagen/pectin 55:45 blend and sterilised by gamma-irradiation.

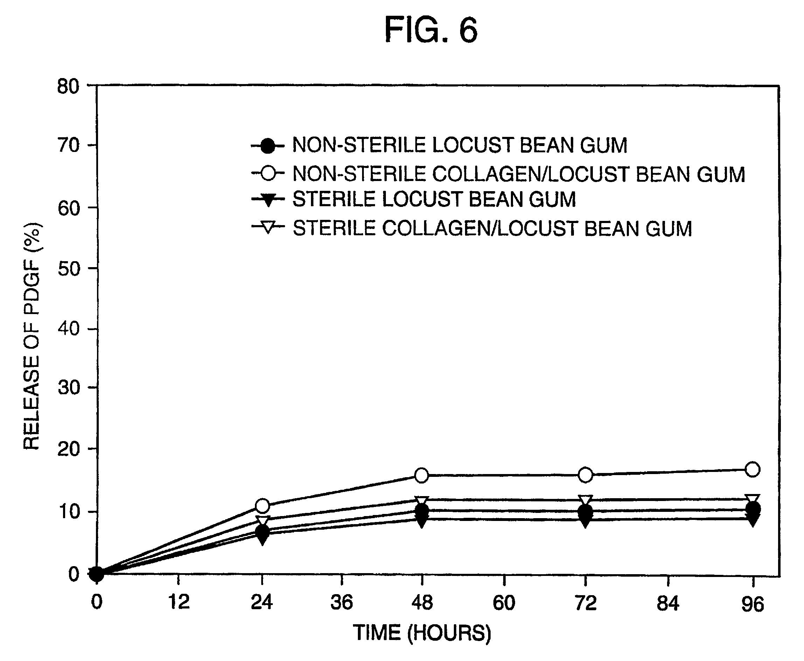

Figure 6 shows a chart of measured PDGF activity in solution against extraction time for the following complexes: (i) PDGF complexed to locust bean gum without gamma irradiation; (ii) PDGF complexed to a collagen/locust bean gum 55:45 mixture; (iii) PDGF complexed to locust bean gum and sterilised by gamma-irradiation; and (iv) PDGF complexed to a collagen/locust bean gum 55:45 blend and sterilised by gamma-irradiation.

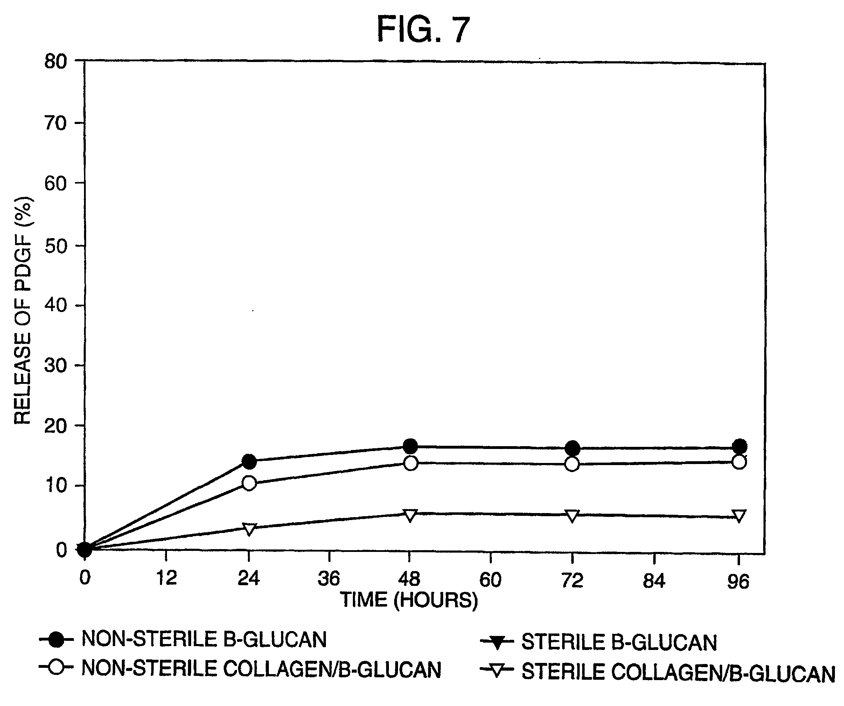

Figure 7 shows a chart of measured PDGF activity in solution against extraction time for the following complexes: (i) PDGF complexed to beta glucan without gamma irradiation; (ii) PDGF complexed to a collagen/beta glucan 55:45 mixture; (iii) PDGF complexed to beta glucan and sterilised by gamma-irradiation; and (iv) PDGF complexed to a collagen/beta glucan 55:45 blend and sterilised by gamma-irradiation.

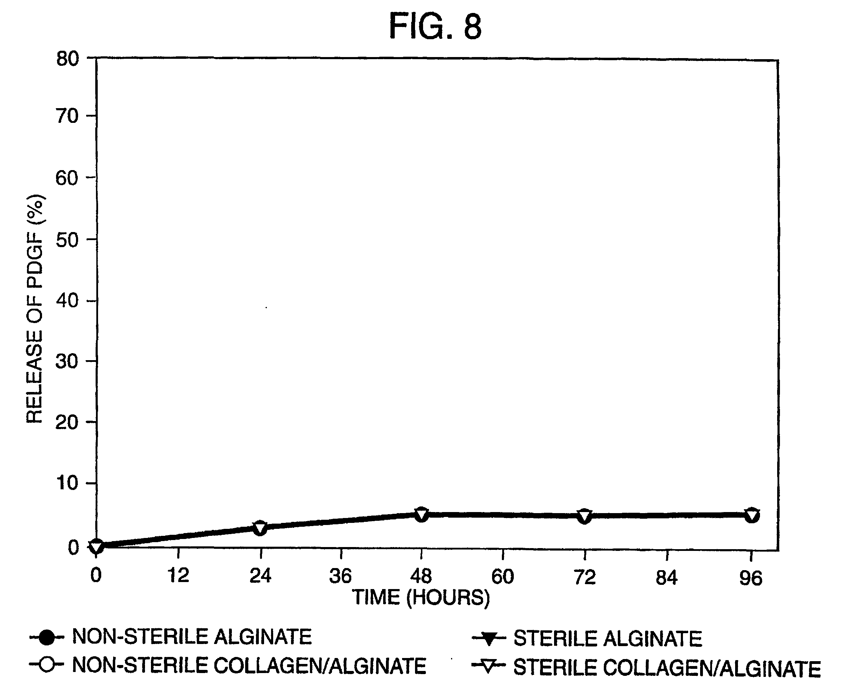

Figure 8 shows a chart of measured PDGF activity in solution against extraction time for the following complexes: (i) PDGF complexed to alginate without gamma irradiation; (ii) PDGF complexed to a collagen/alginate 55:45 mixture; (iii) PDGF complexed to alginate and sterilised by gamma-irradiation; and (iv) PDGF complexed to a collagen/alginate55:45 blend and sterilised by gamma-irradiation.

### Example 1 (Comparative)

A solid sponge of fibrous collagen complexed to platelet derived growth factor (PDGF) was prepared and sterilised as follows.

An aqueous slurry of acid-swollen native collagen fibres derived from the bovine corium was prepared as described in US-A-4614794 or US-A-4320201, the entire contents of which are expressly incorporated herein by reference. The solids content of the slurry was 1 wt.%. The slurry was acidified with 0.05M acetic acid to swell the collagen fibres. Then PDGF (Sigma Chemical Co.) was added to samples of the slurry at concentrations of 0 wt.% (control), 0.1 wt.%, 0-33 wt.% and 1 wt.% based on the weight of the collagen. The slurry was degassed. 30 g of each slurry sample was poured into a 100 cm² petri-dish, blast frozen, and then freeze-dried overnight.

The resulting collagen sponges were cut in half, and one half of each sponge was sterilised by irradiation with 2.5 kGy of ⁶⁰Co gamma-irradiation. The other half of each sponge was left non-sterile as a control.

The samples were tested for PDGF elution and biological activity as described below in Procedure 1.

Typical results are shown in Figure 2. It can be seen that the activity of the PDGF complexed to collagen alone is substantially unchanged by the sterilisation.

A comparative study of was carried out on pure freeze-dried PDGF. The result, shown in Figure 1, demonstrates the large decrease in PDGF activity when sterilisation is performed on the pure PDGF growth factor.

Further control samples of collagen with no added PDGF showed no activity either before or after sterilisation.

### Example 2

A solid sponge of fibrous collagen/ORC complexed to PDGF was prepared as described in Example 1, but with the addition of 80% by weight (based on the weight of the collagen) of milled SURGIGEL® ORC fibres to the collagen slurry before addition of the PDGF. The resulting products are collagen/ORC sponges containing 0(comparative), 0.1, 0.33, and 1.0 wt.% of PDGF based on the weight of collagen plus ORC.

The samples were tested for PDGF elution and biological activity as described below in Procedure 1.

Typical results are shown in Figure 2. It can be seen that the activity of the PDGF complexed to collagen/ORC alone is substantially unchanged by the sterilisation. Surprisingly, it can be seen that the elution of biologically active PDGF from the sterilised complex is even better than the elution from the non-sterile complex.

### Example 3

A solid matrix of ORC coated with PDGF was prepared as follows.
A SURGICEL® ORC cloth (2 cm²) was dipped in a 1% aqueous solution of PDGF, dried at room temperature, and cut in half. One half was sterilised by gamma-irradiation as described in Example 1, and the other half was kept for comparison.

The samples were tested for PDGF elution and biological activity as described below in Procedure 1.

Typical results are shown in Figure 2. It can be seen that the biological activity of the PDGF complexed to ORC alone is substantially unchanged by the sterilisation. Furthermore, the PDGF is more readily eluted from the ORC complex than from the collagen complex, both before and after sterilisation.

### Example 4

A solid sponge of collagen/ORC complexed to aprotinin was prepared as described in Example 2, but with omission of PDGF and the addition of bovine lung aprotinin (Sigma Chemical Co.) in an amount of 0 wt.% (control) and 10 wt.% based on the weight of collagen + ORC.

The complexes were tested for aprotinin binding and activity as described in Procedure 2. The results showed that a substantial fraction of the aprotinin activity was preserved after gamma-irradiation, when the aprotinin was complexed to collagen/ORC.

Surprisingly, the results also showed that the aprotinin was released more rapidly from the irradiated collagen/ORC matrix than from the non-irradiated collagen/ORC matrix.

### Example 5

The ability of chitosan to bind to peptides reversibly and stabilise the peptides against loss of biological activity on sterilisation was investigated as follows.

Solid sponges of chitosan and 55/45 collagen/chitosan having PDGF complexed thereto were prepared as described in Examples 1 and 2, but with replacement of the collagen in Example 1 by chitosan, and with replacement of the ORC in Example 2 by of chitosan in an amount of 45% by weight based on the weight of the complex

Samples of the complexes were submitted to sterilisation by gamma irradiation as described in Example 1.

Procedure 1 was then carried out on the samples to evaluate the amount of biologically active PDGF extracted from the sample by a serum solution as a function of time. Data were collected for both sterile and unsterilised samples.

The results illustrated in Fig. 3 show that a substantial fraction of the original PDGF is released from both complexes both before and after sterilisation. The PDGF is released slightly more readily from the chitosan than from the collagen/chitosan complex. The biological activity of the PDGF is unaffected by sterilisation in the presence of chitosan or collagen/chitosan.

### Example 6 (comparative)

The ability of sodium hyaluronate to bind to peptides reversibly and stabilise the peptides against loss of biological activity on sterilisation was investigated as described above in Example 5, but with replacement of the chitosan of Example 5 by sodium hyaluronate.

The results from Procedure 1 are shown in Fig. 4. It can be seen that the PDGF is readily released from the complexes both before and after sterilisation. However, there is a marked reduction of biological activity of the extracted PDGF after sterilisation in the presence of sodium hyaluronate. The reduction of biological activity is less marked for the collagen/sodium hyaluronate complexes, because the collagen has some stabilising effect on the PDGF as noted above in Example 1. It can be concluded that sodium hyaluronate does not stabilise PDGF against loss of biological activity during sterilisation.

### Example 7 (comparative)

The ability of pectin to bind to peptides reversibly and stabilise the peptides against loss of biological activity on sterilisation was investigated as described above in Example 5, but with replacement of the chitosan of Example 5 by pectin.

The results from Procedure 1 are shown in Fig. 5. It can be seen that the PDGF is readily released from the complexes both before and after sterilisation. However, there is a marked reduction of biological activity of the extracted PDGF after sterilisation in the presence of pectin. The reduction of biological activity is less marked for the collagen/pectin complexes, because the collagen has some stabilising effect on the PDGF as noted above in Example 1. It can be concluded that pectin does not stabilise PDGF against loss of biological activity during sterilisation.

### Example 8

The ability of locust bean gum to bind to peptides reversibly and stabilise the peptides against loss of biological activity on sterilisation was investigated as described above in Example 5, but with replacement of the chitosan of Example 5 by locust bean gum.

The results from Procedure 1 are shown in Fig. 6. It can be seen that relatively little of the biological activity of the PDGF is released from the complexes either before and after sterilisation. However, there is relatively little reduction of biological activity of the extracted PDGF after sterilisation in the presence of locust bean gum. Furthermore, it can be seen that the locust bean gum gives a stabilising effect over and above the effect due to any collagen present in the complexes. It can be concluded that locust bean gum stabilises PDGF against loss of biological activity during sterilisation.

### Example 9 (comparative)

The ability of beta glucan to bind to peptides reversibly and stabilise the peptides against loss of biological activity on sterilisation was investigated as described above in Example 5, but with replacement of the chitosan of Example 5 by beta glucan.

The results from Procedure 1 are shown in Fig. 7. It can be seen that relatively little of the PDGF activity is released from the complexes both before and after sterilisation. Furthermore, there is a marked reduction of biological activity of the extracted PDGF after sterilisation in the presence of beta glucan. It can be concluded that beta glucan does not stabilise PDGF against loss of biological activity during sterilisation.

### Example 10 (comparative)

The ability of sodium alginate to bind to peptides reversibly and stabilise the peptides against loss of biological activity on sterilisation was investigated as described above in Example 5, but with replacement of the chitosan of Example 5 by sodium alginate.

The results from Procedure 1 are shown in Fig. 8. It can be seen that the PDGF is strongly bound to the complexes both before and after sterilisation. The binding is so strong that it is not clear from the released PDGF whether alginate is stabilising the PDGF against loss of biological activity on sterilisation. It can be concluded that sodium alginate is unlikely to be useful to stabilise PDGF against loss of biological activity during sterilisation.

### Example 11

A sterile pharmaceutical gel for topical administration to promote wound healing was formulated as follows (percentages in wt. %):-

| | |
|---|---|
| Carboxymethyl Cellulose | 2.4% |
| Hydroxyethyl Cellulose | 0.3% |
| Sodium chloride | 0.24% |
| Propylene glycol | 20.2% |
| Collagen/ORC/PDGF 1 wt.% | 2.0% |
| Water | balance |

The Collagen/ORC/1 wt.% PDGF was prepared as described in Example 2, comminuted to a particle size of about 1-10µm, sterilised by gamma-irradiation, and then mixed with the other components under aseptic conditions to give a sterile aqueous gel suitable for application to wounds to promote wound healing.

### Example 12

A sterile, substantially pure PDGF was prepared as follows.

The 1% PDGF/collagen sponge prepared in Example 1 was milled to particle size less than 10µm, then sterilised by gamma-irradiation. The sterile complex was treated with sterile saline (pH 8) for 1 hour at 35°C to extract the sterile PDGF, followed by filtration and freeze-drying under aseptic conditions to obtain sterile PDGF.

### Procedure 1

The effects of complexation and sterilisation on the activity of PDGF were assessed as follows:-

A 24-hour releasate of each sample was prepared by placing punch biopsies (6mm diameter) in 1ml Dulbecco's Modified Eagle's Medium (DMEM) and incubating at 37°C, 5% CO₂ in a humidified atmosphere cell culture incubator. Each sample eluant was tested in duplicate.

The biological activity of PDGF was assessed by proliferation using a methylene blue assay to quantify cell number. Briefly, AHDF (adult human dermal fibroblasts) were grown to 95% confluency, trypsinised, counted and replated in 10% FBS/DMEM at a cell density of 3 x 10⁴ cells/ml in a 96 well microtitre plate (100µl/well -3000 cells/well). The cells were allowed to adhere and spread overnight in the humidified atmosphere cell culture incubator at 37°C, 5% CO₂ in this medium. The medium was then removed, the cell monolayer washed with PBS, and the test sample or standard was added at 100µl/well. Each test sample was tested x8 and sterility was achieved by filtering through a syringe filter (0.2µm). Each sample was diluted depending on the estimated concentration of the PDGF incorporated; this was necessary to enable quantification of PDGF over a linear range. Human recombinant PDGF-BB was used as a standard, diluted in SF-DMEM and used over a concentration range 500ng/ml - 1ng/ml. The stimulant was incubated with the cells for a further 3 days at 37°C, 5% CO₂, after which the medium was removed and the cell monolayer fixed with FORMOL® saline. The cells were then stained with methylene blue, excess stain removed and the dye eluted from the cell monolayer with acidified ethanol. The solubilised dye was quantified at 630nm using a microtitre plate spectrophotometer and the data analysed using BIOLINX® software.

The total level of PDGF released from each sample type was determined by ELISA. A monoclonal capture antibody to PDGF-BB was used at 0.5µg/ml 0.025 M-NaHCO₃, 0.025M-Na₂CO₃, 0.025M-Na₂CO₃, pH 9.7 (100µl/well) and incubated overnight at 4°C. Remaining binding sites were blocked with 3% BSA/PBS for 1 hour 37°C after which the test sample or standard was added. Each sample was tested in triplicate and the standard curve ranged from 500ng/ml to 1ng/ml. Each sample was tested at various dilutions to allow an accurate estimation of concentration. The level of PDGF present in the test sample was quantified using a primary polyclonal antibody to PDGF (1/1000 dilution) and a secondary anti-rabbit IgG with a peroxidase conjugate (1/5000 dilution). The level of peroxidase was then quantified using a soluble substrate TMB initially forming a blue colour that turns yellow upon the addition of IM-sulphuric acid. The colour was monitored at 450nm spectrophotometrically.

### Procedure 2

The effects of complexation and sterilisation on the activity of aprotinin were assessed as follows.

3mm punch biopsies were taken in duplicate from collagen/ORC, collagen/ORC containing aprotinin (irradiated), and collagen/ORC containing aprotinin. Two punch biopsies from each sponge were added to 1 ml of DMEM in a sterile plastic container. The DMEM was removed after a number of timepoints ranging from zero - 48hrs incubation at 37°C. The releasates were stored at -20°C until required for the functional assay of aprotinin activity.

The effect of irradiation on the aprotinin functional activity was tested by measuring the ability of aprotinin to inhibit a sample of purified plasmin. Briefly, 10µls of releasate was added to a total assay volume of 100µls, containing 40µls of plasmin (40ng), 10µM plasmin fluorogenic substrate and Tris-HCl buffer (pH 8.1) containing 0.5% TRITON@. Positive controls were run in the absence of releasate.

In addition, a standard curve of plasmin inhibition over a variety of aprotinin concentrations from 0.5ng/ml - 10ng/ml was constructed and used as the basis for calculations.

The assay was monitored over 1hr at 37°C and the amount of fluorescence detected using a fluorogenic plate reader (emission 455nm, excitation 383nm).

The above examples have been disclosed for the purpose of illustration only. Many other embodiments of the present invention falling within the scope of the accompanying drawings will be apparent to the skilled reader.

## Claims

1. A sterile composition comprising a complex of a therapeutic peptide and a polysaccharide selected from the group consisting of cellulose derivatives, chitin, chitosans, galactomannans, and mixtures thereof, wherein said complex has been sterilised with ionising radiation.

2. A sterile composition according to claim 1, wherein said peptide is selected from the group consisting of growth factors, haemostatic agents, antimicrobial agents, antibacterial agents, anti-adhesion agents and collagen fragments.

3. A sterile composition according to claim 2, wherein the peptide comprises a growth factor having human mitogenic or angiogenic activity.

4. A sterile composition according to claim 3, wherein the growth factor is selected from the group consisting of fibroblast growth factor (FGF), Platelet derived growth factor (PDGF), transforming growth factors (TGF-α, TGF-β₁, TGF-β₂), nerve growth factors (NGF-α, NGF-β), epidermal growth factor (EGF), bone morphogenetic protein, insulin-like growth factors (IGF-I or IGF-II), and mixtures thereof.

5. A sterile composition according to any preceding claim, wherein said composition further comprises a biopolymer selected from the group consisting of structural proteins, polyanionic polysaccharides, and mixtures thereof.

6. A sterile composition according to claim 5, wherein said structural proteins are selected from the group consisting of native-collagen types, atelocollagen, pepsin-solubilized collagen, gelatin, fibronectin and laminin.

7. A sterile composition according to claim 6, wherein said structural proteins consist essentially of native fibrous collagen.

8. A sterile composition according to any preceding claim, wherein said polysaccharide is selected from the group consisting of oxidized celluloses, chitosans, and salts and mixtures thereof.

9. A sterile composition according to claim 8, wherein said polysaccharide is selected from the group consisting of ORC and nORC.

10. A sterile composition according to any preceding claim, wherein said composition comprises a mixture of native fibrous collagen, and ORC.

11. A sterile composition according to any preceding claim, wherein the weight ratio of said therapeutic peptide to said polysaccharide is from 1:10⁶ to 1:10.

12. A sterile composition according to claim 11, wherein said weight ratio of from 1 to 10⁵ to 1:100.

13. A sterile composition according to claim 12, wherein said weight ratio is from 1 to 10⁴ to 1:1000.

14. A sterile composition according to any preceding claim, wherein said composition comprises a freeze-dried or solvent dried sponge.

15. A sterile composition according to claim 14, further comprising a free radical scavenger.

16. A sterile composition according to any preceding claim, wherein said composition is a viscous liquid or gel for topical administration to the human or animal body.

17. A sterile composition according to any one of claims 1 to 15, wherein said composition is a liquid for intravenous administration to the human or animal body.

18. A sterile composition according to any one of claims 1 to 13, wherein said therapeutic peptide and polysaccharide are coated onto a surface of a solid carrier.

19. A sterile composition according to claim 18, wherein said solid carrier is a woven or nonwoven fabric, a polymer film, or a web, suitable for application to a surface of a wound.

20. A sterile composition according to claim 18 or 19, wherein said solid carrier is bioabsorbable.

21. A sterile composition according to claim 20, wherein said solid carrier comprises a woven or nonwoven ORC cloth.

22. A sterile composition according to any preceding claim which is sterile packaged.

23. A process for the preparation of a sterile therapeutic composition comprising the steps of:
providing a complex of a therapeutic peptide with a polysaccharide; and
sterilizing said complex; followed by
dispersing said complex in or on a pharmaceutically acceptable carrier,
said polysaccharide being selected from the group consisting of cellulose derivatives, chitin, chitosans, galactomannans, and mixtures thereof.

24. A process according to claim 23, wherein said step of providing comprises mixing said therapeutic peptide with said polysaccharide in a solvent, followed by removing said solvent to leave said complex.

25. A process according to claim 24, wherein said solvent is an aqueous solvent.

26. A process according to claim 24 or 25, wherein said solvent is removed by freeze drying or solvent drying.

27. A process according to any one of claims 23 to 26 wherein said step of sterilizing is carried out with ionizing radiation or by treatment with a gas plasma.

28. A process according to claim 27, wherein said ionizing radiation is gamma radiation.

29. A process according to any one of claims 23 to 28, wherein said complex comprises less than 4% by weight of water during said step of sterilizing.

30. A process according to any one of claims 23 to 29, wherein said step of dispersing is carried out under aseptic conditions, after said step of sterilizing.

31. A process according to any one of claims 23 to 30 for the preparation of a sterile composition according to any one of claims 1 to 22.

32. A process for the preparation of a sterile therapeutic peptide comprising the steps of:
providing a therapeutic peptide;
forming a complex between the therapeutic peptide and a polysaccharide;
sterilizing said complex; followed by
separating the therapeutic peptide from said complex under aseptic conditions to obtain said sterile therapeutic peptide, said polysaccharide being selected from the group consisting of cellulose derivatives, chitin, chitosans, galactomannans, and mixtures thereof.

33. A process according to claim 32, wherein said step of separating comprises extracting the therapeutic peptide from the complex with an aqueous solvent.

## Patentansprüche

1. Sterile Zusammensetzung, umfassend einen Komplex eines therapeutischen Peptids und ein Polysaccharid, ausgewählt aus der Gruppe bestehend aus Zellulose-Derivaten, Chitin, Chitosanen, Galactomannanen und Gemischen davon, wobei der Komplex durch ionisierende Strahlung sterilisiert wurde.

2. Sterile Zusammensetzung nach Anspruch 1, wobei das Peptid ausgewählt ist aus der Gruppe bestehend aus Wachstumsfaktoren, hämostatischen Mitteln, antimikrobiellen Mitteln, antibakteriellen Mitteln, anti-Adhäsionsmitteln und Kollagen-Fragmenten.

3. Sterile Zusammensetzung nach Anspruch 2, wobei das Peptid einen Wachstumsfaktor umfaßt, der menschliche mitogene oder angiogenetische Aktivität aufweist.

4. Sterile Zusammensetzung nach Anspruch 3, wobei der Wachstumsfaktor ausgewählt ist aus der Gruppe bestehend aus Fibroblasten-Wachstumsfaktor (FGF), Plättchenabgeleiteter Wachstumsfaktor (PDGF), transformierende Wachstumsfaktoren (TGF-α, TGF-β₁, TGF-β₂), Nervenwachstumsfaktoren (NGF-α, NGF-β), epidermalem Wachstumsfaktor (EGF), Knochen-morphogenetischen Proteinen, Insulin-ähnlichen Wachstumsfaktoren (IGF-I oder IGF-II) und Gemischen davon.

5. Sterile Zusammensetzung nach einem der voranstehenden Ansprüche, wobei die Zusammensetzung weiterhin ein Biopolymer umfaßt, ausgewählt aus der Gruppe bestehend aus strukturellen Proteinen, polyanionischen Polysacchariden und Gemischen davon.

6. Sterile Zusammensetzung nach Anspruch 1, wobei die strukturellen Proteine ausgewählt sind aus der Gruppe bestehend aus nativ-Kollagen-Typen, Atelokollagen, Pepsin-solubisiertem Kollagen, Gelatine, Fibronektin und Laminin.

7. Sterile Zusammensetzung nach Anspruch 6, wobei die strukturellen Proteine im wesentlichen aus nativem fibrösem Kollagen bestehen.

8. Sterile Zusammensetzung nach einem der voranstehenden Ansprüche, wobei das Polysaccharid ausgewählt ist aus der Gruppe bestehend aus oxidierten Zellulosen, Chitosanen und Salzen und Gemischen davon.

9. Sterile Zusammensetzung nach Anspruch 8, wobei das Polysaccharid ausgewählt ist aus der Gruppe bestehend aus ORC und nORC.

10. Sterile Zusammensetzung nach einem der voranstehenden Ansprüche, wobei die Zusammensetzung ein Gemisch von nativem fibrösem Kollagen und ORC umfaßt.

11. Sterile Zusammensetzung nach einem der voranstehenden Ansrpüche, wobei das Gewichtsverhältnis des therapeutischen Peptids zu dem Polysaccharid von 1:10⁶ bis 1:10 ist.

12. Sterile Zusammensetzung nach Anspruch 11, wobei das Gewichtsverhältnis von 1:10⁵ bis 1:100 ist.

13. Sterile Zusammensetzung nach Anspruch 12, wobei das Gewichtsverhältnis von 1:10⁴ bis 1:1000 ist.

14. Sterile Zusammensetzung nach einem der voranstehenden Ansprüche, wobei die Zusammensetzung einen gefriergetrockneten oder Lösungsmittel-getrockneten Schwamm umfaßt.

15. Sterile Zusammensetzung nach Anspruch 14, weiter umfassend einen freie Radikale-Fänger.

16. Sterile Zusammensetzung nach einem der voranstehenden Ansprüche, wobei die Zusammensetzung eine viskose Flüssigkeit oder Gel zur topischen Verabreichung auf dem menschlichen oder tierischen Körper ist.

17. Sterile Zusammensetzung nach einem der Ansprüche 1 bis 15, wobei die Zusammensetzung eine Flüssigkeit zur intravenösen Verabreichung an den menschlichen oder tierischen Körper ist.

18. Sterile Zusammensetzung nach einem der Ansprüche 1 bis 13, wobei das therapeutische Peptid und das Polysaccharid auf eine Oberfläche eines festen Trägers beschichtet sind.

19. Sterile Zusammensetzung nach Anspruch 18, wobei der feste Träger ein gewobenes oder nicht gewobenes Gewebe, ein Polymerfilm oder ein Netz ist, das für die Verabreichung auf eine Oberfläche einer Wunde geeignet ist.

20. Sterile Zusammensetzung nach Anspruch 18 oder 19, wobei der feste Träger bioabsorptionsfähig ist.

21. Sterile Zusammensetzung nach Anspruch 20, wobei der feste Träger einen gewobenen oder nicht gewobenen ORC-Stoff umfaßt.

22. Sterile Zusammensetzung nach einem der voranstehenden Ansprüche, die steril verpackt ist.

23. Verfahren zur Herstellung einer sterilen therapeutischen Zusammensetzung, umfassend die Schritte von:
zur Verfügung Stellen eines Komplexes eines therapeutischen Peptids mit einem Polysaccharid; und
Sterilisieren des Komplexes; gefolgt von
Dispergieren des Komplexes in oder auf einem pharmazeutisch akzeptablen Träger,
wobei das Polysaccharid ausgewählt ist aus der Gruppe bestehend aus Zellulosederivaten, Chitin, Chitosanen, Galaktomannanen und Gemischen davon.

24. Verfahren nach Anspruch 23, wobei der Schritt des zur Verfügung Stellens ein Mischen des therapeutischen Peptids mit dem Polysaccharid in einem Lösungsmittel umfaßt, gefolgt vom Entfernen des Lösungsmittels, um den Komplex freizusetzen.

25. Verfahren nach Anspruch 24, wobei das Lösungsmittel ein wäßriges Lösungsmittel ist.

26. Verfahren nach Anspruch 24 oder 25, wobei das Lösungsmittel durch Gefriertrocknen oder Lösungsmitteltrocknen entfernt wird.

27. Verfahren nach einem der Ansprüche 23 bis 26, wobei der Schritt des Sterilisierens mit ionisierender Strahlung oder durch Behandlung mit einem Gasplasma durchgeführt wird.

28. Verfahren nach Anspruch 27, wobei die ionisierende Strahlung Gammastrahlung ist.

29. Verfahren nach einem der Ansprüche 23 bis 28, wobei der Komplex weniger als 4 Gew.-% Wasser während des Schritts der Sterilisierung umfaßt.

30. Verfahren nach einem der Ansprüche 23 bis 29, wobei der Schritt des Dispergierens unter aseptischen Bedingungen durchgeführt wird, nach dem Schritt der Sterilisierung.

31. Verfahren nach einem der Ansprüche 23 bis 30 zur Herstellung einer sterilen Zusammensetzung nach einem der Ansprüche 1 bis 22.

32. Verfahren zur Herstellung eines sterilen therapeutischen Peptids, umfassend die Schritte von:
zur Verfügung Stellen eines therapeutischen Peptids;
Bilden eines Komplexes zwischen dem therapeutischen Peptid und einem Polysaccharid;
Sterilisieren des Komplexes; gefolgt von Trennen des therapeutischen Peptids von dem Komplex unter aseptischen Bedingungen, um so das sterile therapeutische Peptid zu erhalten, wobei das Polysaccharid ausgewählt ist aus der Gruppe bestehend aus Zellulosederivaten, Chitin, Chitosan, Galactomannanen und Gemischen davon.

33. Verfahren nach Anspruch 32, wobei der Schritt des Abtrennens ein Extrahieren des therapeutischen Peptids aus dem Komplex mit einem wäßrigen Lösungsmittel umfaßt.

## Revendications

1. Composition stérile comprenant un complexe d'un peptide thérapeutique et d'un polysaccharide choisi parmi le groupe constitué par les dérivés de la cellulose, la chitine, les chitosanes, les galactomannanes et leurs mélanges, dans laquelle ledit complexe a été stérilisé par des radiations ionisantes.

2. Composition stérile selon la revendication 1, dans laquelle ledit peptide est choisi parmi le groupe constitué par les facteurs de croissance, les agents hémostatiques, les agents antimicrobiens, les agents antibactériens, les agents anti-adhésion et les fragments de collagène.

3. Composition stérile selon la revendication 2, dans laquelle le peptide comprend un facteur de croissance ayant une activité mitogène ou angiogène chez l'homme.

4. Composition stérile selon la revendication 3, dans laquelle le facteur de croissance est choisi parmi le groupe constitué par le facteur de croissance des fibroblastes (FGF), le facteur de croissance plaquettaire (PDGF), les facteurs de croissance transformants (TGF-α, TGF-β₁, TGF-β₂), les facteurs de croissance nerveux (NGF- α et NGF-β), le facteur de croissance épidermique (EGF), la protéine morphogénétique des os, les facteurs de croissance de type insuline (IGF-I ou IGF-II), et leurs mélanges.

5. Composition stérile selon l'une des revendications précédentes, dans laquelle ladite composition comprend en outre un biopolymère choisi parmi le groupe constitué par les protéines structurales, les polysaccharides polyanioniques, et leurs mélanges.

6. Composition stérile selon la revendication 5, dans laquelle lesdites protéines structurales sont choisies parmi le groupe constitué par les types collagène-natif, atélocollagène, collagène solubilisé par la pepsine, gélatine, fibronectine et laminine.

7. Composition stérile selon la revendication 6, dans laquelle lesdites protéines structurales sont essentiellement constituées par du collagène fibreux natif.

8. Composition stérile selon l'une quelconque des revendications précédentes, dans laquelle ledit polysacharide est choisi parmi le groupe constitué par les celluloses oxydées, les chitosanes, et leurs sels et leurs mélanges.

9. Composition stérile selon la revendication 8, dans laquelle ledit polysacharide est choisi parmi le groupe constitué par ORC et nORC.

10. Composition stérile selon l'une quelconque des revendications précédentes, dans laquelle ladite composition comprend un mélange de collagène natif fibreux, et d'ORC.

11. Composition stérile selon l'une quelconque des revendications précédentes, dans laquelle le ratio pondéral dudit peptide thérapeutique par rapport audit polysaccharide est compris entre 1:10⁶ et 1:10.

12. Composition stérile selon la revendication 11, dans laquelle ledit ratio pondéral est compris entre 1:10⁵ jusqu'à 1:100.

13. Composition stérile selon la revendication 12, dans laquelle ledit ration pondéral est de 1:10⁴ à 1:1000.

14. Composition stérile selon l'une quelconque des revendications précédentes, dans laquelle ladite composition comprend une éponge lyophilisée ou séchée par un solvant.

15. Composition stérile selon la revendication 14, comprenant en outre un capteur de radicaux libres.

16. Composition stérile selon l'une quelconque des revendications précédentes, dans laquelle ladite composition est un liquide visqueux ou un gel pour l'administration topique sur le corps humain ou d'un animal.

17. Composition stérile selon l'une quelconque des revendications 1 à 15, dans laquelle ladite composition est un liquide pour l'administration intraveineuse dans le corps humain ou d'un animal.

18. Composition stérile selon l'une quelconque des revendications 1 à 13, dans laquelle ledit peptide thérapeutique et le polysaccharide sont revêtus sur une surface d'un véhicule solide.

19. Composition stérile selon la revendication 18, dans laquelle ledit véhicule solide est un tissu tissé ou non-tissé, un film de polymère, ou un réseau, convenant pour l'application à la surface d'une blessure.

20. Composition stérile selon la revendication 18 ou 19, dans laquelle ledit véhicule solide est bioabsorbable.

21. Composition stérile selon la revendication 20, dans laquelle ledit véhicule solide comprend une étoffe ORC tissée ou non tissée.

22. Composition stérile selon l'une quelconque des revendications précédentes qui est emballée stérilement.

23. Procédé de préparation d'une composition thérapeutique stérile comprenant les étapes consistant à:
- fournir un complexe d'un peptide thérapeutique avec un polysaccharide; et
- stériliser ledit complexe; puis
- disperser ledit complexe dans ou sur un véhicule pharmaceutiquement acceptable, ledit polysaccharide étant choisi parmi le groupe constitué par les dérivés de la cellulose, la chitine, les chitosanes, les galactomannanes et leurs mélanges.

24. Procédé selon la revendication 23, dans lequel ladite étape de fourniture comprend le mélange dudit peptide thérapeutique avec ledit polysacharide dans un solvant, suivi par l'élimination dudit solvant pour obtenir le complexe.

25. Procédé selon la revendication 24, dans lequel ledit solvant est un solvant aqueux.

26. Procédé selon la revendication 24 ou 25, dans lequel ledit solvant est éliminé par lyophilisation ou séchage par solvant.

27. Procédé selon l'une quelconque des revendications 23 à 26, dans lequel ladite étape de stérilisation est réalisée avec des radiations ionisantes ou par traitement avec un plasma de gaz.

28. Procédé selon la revendication 27, dans lequel ladite radiation ionisante est une radiation gamma.

29. Procédé selon l'une quelconque des revendications 23 à 28, dans lequel ledit complexe comprend moins de 4% en poids d'eau pendant ladite étape de stérilisation.

30. Procédé selon l'une quelconque des revendications 23 à 29, dans lequel ladite étape de dispersion est réalisée dans des conditions aseptiques, après ladite étape de stérilisation.

31. Procédé selon l'une quelconque des revendications 23 à 30 pour la préparation d'une composition stérile selon l'une quelconque des revendications 1 à 22.

32. Procédé de préparation d'un peptide thérapeutique stérile comprenant les étapes consistant à:
- fournir un peptide thérapeutique;
- former un complexe entre le peptide thérapeutique et un polysaccharide;
- stériliser ledit complexe; puis
- séparer le peptide thérapeutique dudit complexe dans des conditions aseptiques pour obtenir ledit peptide thérapeutique stérile, ledit polysaccharide étant choisi parmi le groupe constitué par les dérivés de la cellulose, la chitine, les chitosanes, les galactomannanes et leurs mélanges.

33. Procédé selon la revendication 32, dans lequel ladite étape de séparation comprend l'extraction du peptide thérapeutique du complexe avec un solvant aqueux.
